# EUROPEAN PATENT APPLICATION

(11) **EP 4 663 622 A1**
(43) Date of publication of application: **17.12.2025**
(21) Application number: 24926776.6
(22) Date of filing: 19.10.2024
(51) Int. Cl.: C07C 29/141, C07C 31/26, C07C 31/18, C07H 15/04, C07H 1/00

(54) **CONTINUOUS GAS-LIQUID COUNTERCURRENT METHOD FOR PREPARING SUGAR ALCOHOL**

(30) Priority: 27.02.2024 CN 202410214665
(71) Applicant: INSTITUTE OF ZHEJIANG UNIVERSITY-QUZHOU, Quzhou, Zhejiang 324000 (CN); Zhejiang Huakang Pharmaceutical Co., Ltd., Quzhou, Zhejiang 324302 (CN)
(72) Inventor: FU, Jie, Quzhou, Zhejiang 324000 (CN); FAN, Haoan, Quzhou, Zhejiang 324000 (CN); LI, Bolong, Quzhou, Zhejiang 324000 (CN); WANG, Jianghao, Quzhou, Zhejiang 324000 (CN); LV, Xiuyang, Quzhou, Zhejiang 324000 (CN); LI, Mian, Santa Clara, CA (US); YANG, Wulong, Quzhou, Zhejiang 324302 (CN); AN, Yanlong, Quzhou, Zhejiang 324302 (CN); LIAO, Chengjun, Quzhou, Zhejiang 324302 (CN)
(74) Representative: Dai, Simin
(86) International application number: PCT/CN2024/125952
(87) International publication number: WO 2025/179914

(57) **Abstract**

Provided is a method for continuous gas-liquid countercurrent preparing sugar alcohol, the method includes: in a gas-liquid countercurrent trickle bed reactor, pouring a biomass sugar solution from a liquid phase inlet into the trickle bed reactor from top to bottom, and introducing hydrogen gas from a gas phase inlet to the trickle bed reactor from bottom to top; the biomass sugar solution and the hydrogen gas pass through a catalyst bed in a gas-liquid countercurrent manner for hydrogenation reduction to obtain a biomass sugar alcohol solution. By highly efficiently intensifying the gas-liquid-solid three-phase mass transfer and shortening the residence time, the method for continuous gas-liquid countercurrent preparing sugar alcohol provided by the present disclosure realizes the continuous hydrogenation of the biomass sugar solution, and substantially improves the conversion rate of the biomass sugar and the yield of the corresponding sugar alcohol.

## Description

### TECHNICAL FIELD

The present disclosure relates to the field of hydrogenation for the production of sugar alcohols, and in particular, to a method for continuous gas-liquid countercurrent preparing sugar alcohols.

### BACKGROUND

Existing sugar alcohols refer to a class of functional polyols made from biomass sugar by hydrogenation and reduction, including maltitol, sorbitol, mannitol, xylitol, lactitol, etc., which are important raw materials and products in industries such as food, fine chemicals, and pharmaceuticals. Compared with common aldoses or ketoses, biomass sugar alcohols have the advantages of low caloric value, low glycemic response, and non-cariogenic properties, making them globally recognized as safe and healthy food products.

At present, the main production process for biomass sugar alcohols is batch hydrogenation in batch reactors, where catalysts are easily lost by mechanical agitation, and the catalysts are difficult to recover. In addition, in order to ensure the thorough hydrogenation of biomass sugar in the industrial production process, it is common to continuously replenish the hydrogen gas to maintain the hydrogen gas supersaturation state in the sugar solution. To address the above problems, the continuous hydrogenation process has been progressively developed and applied in this field. U.S. Patent US8816068B2 discloses a continuous catalytic hydrogenation process for preparing high-purity sugar alcohols, which employs two sets of fixed-bed reactors in series to achieve continuous hydrogenation of glucose. The process has an excellent glucose conversion rate and sorbitol selectivity. However, the catalysts loaded in the reactor are ruthenium-based and platinum-based catalysts, which are relatively expensive. Additionally, the preferred feed flow rate in patent US8816068B2 is 1 kg/L/h-1.5 kg/L/h, a flow rate of the hydrogen gas is 15 kg/h, and a pressure of the hydrogen gas is 8 MPa-15 MPa, which is characterized by a high cost, a high hydrogen consumption, and a high pressure of the hydrogen gas, limiting its industrialization. Patent WO2018118854A1 publishes a method for the preparation of maltitol by continuous hydrogenation of maltose. The method uses a fixed-bed reactor to achieve a high yield (>90%), but the technique has severe limitations in terms of operating conditions, such as the molar ratio of hydrogen gas to maltose (>40:1) and the reaction pressure of hydrogen gas (12.4 MPa- 17.2 MPa). Chinese patent CN208949158U published a device for preparing sorbitol by continuous hydrogenation of glucose, combining a slurry-bed reactor connects with a fixed-bed reactor sequentially, but the total material residence time is long, and the production efficiency is low. In addition, the reaction pressure of the hydrogen gas used in patent CN208949158U is >10.5 MPa, and there are cost and safety issues.

In summary, the prior art still suffers from a large molar ratio of the hydrogen gas to substrate, a high consumption of the hydrogen gas, and a low production efficiency, which need to be improved.

### SUMMARY

The technical objective of the present disclosure is to provide a method for continuous gas-liquid countercurrent preparing sugar alcohol, which realizes the continuous hydrogenation of a biomass sugar solution by highly efficiently intensifying the gas-liquid-solid three-phase mass transfer and shortening the residence time, and substantially improves the conversion rate of the biomass sugar and the yield of the corresponding sugar alcohol.

The technical solution adopted by the present disclosure to achieve the above technical objective is as follows.

A method for continuous gas-liquid countercurrent preparing sugar alcohol, wherein the method includes: in a gas-liquid countercurrent trickle bed reactor, pouring a biomass sugar solution from a liquid phase inlet into the trickle bed reactor from top to bottom; and introducing hydrogen gas from a gas phase inlet to the trickle bed reactor from bottom to top, wherein the biomass sugar solution and the hydrogen gas pass through a catalyst bed in a gas-liquid countercurrent manner for hydrogenation reduction to obtain a biomass sugar alcohol solution.

The catalyst is selected from Raney nickel, Raney copper, ruthenium carbon, or platinum carbon.

Preferably, the catalyst is Raney nickel or Raney copper. The catalyst is relatively inexpensive and has high catalytic efficiency.

An average particle size of the catalyst is in a range of 1 mm-6 mm. The particle size can effectively avoid the problem of pressure drop caused by catalyst particles being too small, while also avoiding uneven dispersion of liquid caused by particles being too large.

The biomass sugar solution includes, but is not limited to, maltose, glucose, mannose, xylose, or lactose. The corresponding sugar alcohol includes, but is not limited to, maltitol, sorbitol, mannitol, xylitol, or lactitol.

A molar concentration of the biomass sugar solution is in a range of 0.6 mol/L -2.2 mol/L. The molar concentration of the biomass sugar solution can effectively enhance the product concentration and reduce the cost of subsequent crystallization and separation on the basis of ensuring the conversion efficiency.

The biomass sugar solution is continuously fed using a liquid-phase pump, with a feed flow rate of the biomass sugar solution in a range of 0.5 mL/min-5 mL/min. The feed flow rate is conducive to prolonging the residence time of the liquid on the surface of the catalyst, enhancing the yield of the sugar alcohol, and effectively preventing the hydrolysis side reaction caused by local overheating.

A pressure of the hydrogen gas is in a range of 5 Mpa-10 MPa. The pressure of the hydrogen gas can ensure the excellent conversion rate of the biomass sugar and the yield of the sugar alcohol, and take into account safety and economic considerations.

The flow rate of the hydrogen gas is controlled using a mass flow meter, and a flow rate of the hydrogen gas is in a range of 10 sccm- 100 sccm.

A temperature of the gas-liquid countercurrent trickle bed reactor is in a range of 100°C-140°C. The temperature of the gas-liquid countercurrent trickle bed reactor can balance high conversion and high selectivity to maximize the generation of target sugar alcohol.

Preferably, the average particle size of the catalyst is in a range of 1 mm-4 mm, the molar concentration of the biomass sugar solution is in a range of 0.6 mol/L -1.8 mol/L, the feed flow rate of the biomass sugar solution is in a range of 1 mL/min-2 mL/min, the pressure of the hydrogen gas is in a range of 6 Mpa-9 MPa, the flow rate of the hydrogen gas is in a range of 20 sccm-80 sccm, and the temperature of the trickle bed reactor is in a range of 120°C-140°C. The above-described process conditions can achieve a conversion rate of the biomass sugar of at least 87% and the yield of the sugar alcohol of at least 83%.

Further preferably, the average particle size of the catalyst is in a range of 1 mm-3.5 mm, the molar concentration of the biomass sugar solution is in a range of 0.6 mol/L -1.6 mol/L, the feed flow rate of the biomass sugar solution is in a range of 1 mL/min-1.5 mL/min, the pressure of the hydrogen gas is in a range of 6 Mpa-9 MPa, the flow rate of the hydrogen gas is in a range of 20 sccm-70 sccm, and the temperature of the trickle bed reactor is in a range of 120°C-140°C. The above-described process conditions can achieve a conversion rate of the biomass sugar of at least 92% and the yield of the sugar alcohol of at least 90%.

Further preferably, the average particle size of the catalyst is in a range of 1 mm-3.5 mm, the molar concentration of the biomass sugar solution is in a range of 1 mol/L -1.6 mol/L, the feed flow rate of the biomass sugar solution is in a range of 1 mL/min-1.5 mL/min, the pressure of the hydrogen gas is in a range of 6 Mpa-9 MPa, the flow rate of the hydrogen gas is in a range of 20 sccm-50 sccm, and the temperature of the trickle bed reactor is in a range of 120°C-140°C. The above-described process conditions can achieve the conversion rate of the biomass sugar of at least 97.5% and the yield of the sugar alcohol of at least 94.5%.

Further preferably, the average particle size of the catalyst is in a range of 2 mm-3 mm, the molar concentration of the biomass sugar solution is in a range of 1.4 mol/L -1.6 mol/L, the feed flow rate of the biomass sugar solution is 1 mL/min, the pressure of the hydrogen gas is in a range of 6 Mpa-8 MPa, the flow rate of the hydrogen gas is in a range of 20 sccm-50 sccm, and the temperature of the trickle bed reactor is in a range of 120°C-130°C. The above-described process conditions can achieve a conversion rate of the biomass sugar of at least 99% and the yield of the sugar alcohol of at least 98%.

Compared with the prior art, the beneficial effects of the present disclosure are:
(1) Compared with the existing continuous hydrogenation process of biomass sugar, the present disclosure adopts a gas-liquid countercurrent trickle bed reactor to prepare the corresponding sugar alcohol, which can effectively ensure the full contact among the hydrogen gas, the biomass sugar solution, and the catalyst. By strengthening the three-phase mass transfer, the residence time of the material is shortened, thereby improving the production efficiency of sugar alcohol.
(2) The pressure and the flow rate of the hydrogen gas of the present disclosure are relatively low, and the amount of hydrogen gas is relatively small, which greatly reduces the production cost and effectively avoids the safety hazards caused by high-pressure hydrogen gas.
(3) The present disclosure applies to the continuous hydrogenation of a plurality of biomass sugars, and can maintain a good conversion rate of the biomass sugar and a good yield of the sugar alcohol (including space-time yield) under a high concentration material condition (such as 1.2 mol/L-1.8 mol/L), which has excellent industrialization and scaling-up potential.
(4) The present disclosure can be widely used in the industrialized preparation of functional sugar alcohols such as maltitol, sorbitol, mannitol, xylitol, lactitol, or the like.

### BRIEF DESCRIPTION OF THE DRAWINGS

FIG. 1 is a schematic structure diagram of a gas-liquid countercurrent trickle bed reactor of the present disclosure.
   Markings in FIG. 1 denote: 11, liquid phase inlet; 12, gas phase outlet; 13, gas phase inlet; 14, liquid phase outlet; 15, upper layer of quartz sand filler; 16, catalyst bed; 17, lower layer of quartz sand filler; 18, support frame.

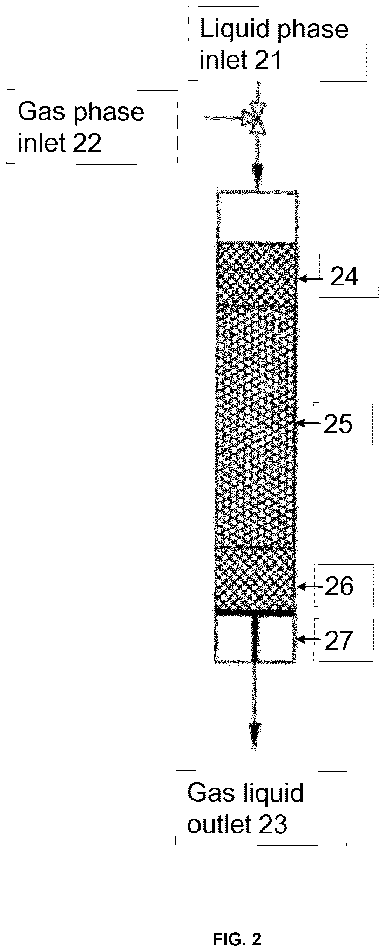

FIG. 2 is a schematic structure diagram of a gas-liquid concurrent trickle bed reactor in comparative example 1 of the present disclosure.
   Markings in FIG. 2 denote: 21, liquid phase inlet; 22, gas phase inlet; 23, gas liquid outlet; 24, upper layer of quartz sand filler; 25, catalyst bed; 26, lower layer of quartz sand filler; 27, support frame.

### DETAILED DESCRIPTION

In order to make the above objectives, features, and advantages of the present disclosure more obvious and understandable, the following specific embodiments of the present disclosure are described in detail in conjunction with the embodiments.

Many specific details are set forth in the following description in order to facilitate a full understanding of the present disclosure, but the present disclosure may be practiced in other ways than those described herein, and a person skilled in the art may do similarly without violating the connotation of the present disclosure. Therefore, the present disclosure is not limited by the embodiments disclosed below.

Secondly, the term "one embodiment" or "embodiments" referred to herein refers to specific features, structures, or characteristics that may be included in at least one implementation of the present disclosure. The term 'in one embodiment' appearing in different places in this specification does not necessarily refer to the same embodiment, nor is it a separate or selective embodiment that is mutually exclusive with other embodiments.

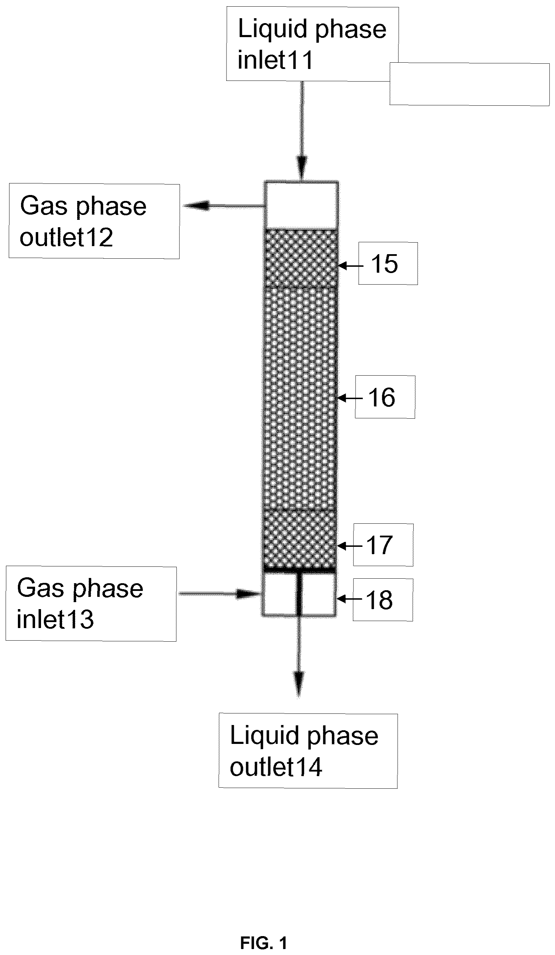

As shown in FIG. 1, the present disclosure provides a method for continuous gas-liquid countercurrent preparing sugar alcohol. In a gas-liquid countercurrent trickle bed reactor, a catalyst bed is loaded on top of a support frame 18. A biomass sugar solution flows into the reactor from a liquid phase inlet 11 from top to bottom, and hydrogen gas flows into the reactor from a gas phase inlet 13 from bottom to top. The biomass sugar solution and the hydrogen gas pass through an upper layer of quartz sand filler 15, a catalyst bed 16, and a lower layer of quartz sand filler 17 in a gas-liquid countercurrent manner for hydrogenation reduction, resulting in a biomass sugar alcohol solution.

### Example 1:

The gas-liquid countercurrent trickle bed reactor was filled with 90 g of Raney nickel catalyst (with an average particle size of 1 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from a gas phase outlet 12 at the upper end of the reactor, and the pressure inside the reactor was controlled by a back-pressure valve at 8 MPa. Meanwhile, a liquid-phase metering pump was used to add 1.4 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 97.5%, and a yield of the maltitol is 94.8%.

### Example 2:

The gas-liquid countercurrent trickle bed reactor was filled with 90 g of Raney nickel catalyst (with an average particle size of 3 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 8 MPa. Meanwhile, the liquid-phase metering pump was used to add 1.4 mol/L maltose solution to the reactor from the liquid phase inlet 11 at the top end of the reactor at a flow rate of 3 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 70.4%, and a yield of the maltitol is 67.9%.

### Example 3:

The gas-liquid countercurrent trickle bed reactor was filled with 90 g of Raney nickel catalyst (with an average particle size of 4 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 8 MPa. Meanwhile, the liquid-phase metering pump was used to add 1.4 mol/L maltose solution to the reactor from the liquid phase inlet 11 at the top end of the reactor at a flow rate of 5 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 55.1%, and a yield of the maltitol is 53.5%.

### Example 4:

The gas-liquid countercurrent trickle bed reactor was filled with 80 g of Raney nickel catalyst (with an average particle size of 3 mm), the reactor was heated up to 110 °C, the air inside the reactor was replaced and expelled with high-purity nitrogen, and then 50 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 7.5 MPa. Meanwhile, the liquid-phase metering pump was used to add 1.4 mol/L maltose solution to the reactor from the liquid phase inlet 11 at the top end of the reactor at a flow rate of 1.5 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 75.5%, and a yield of the maltitol is 69.3%.

### Example 5:

The gas-liquid countercurrent trickle bed reactor was filled with 80 g of Raney nickel catalyst (with an average particle size of 3 mm), the reactor was heated up to 120 °C, the air inside the reactor was replaced and expelled with high-purity nitrogen, and then 50 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 7.5 MPa. Meanwhile, the liquid-phase metering pump was used to add 1.4 mol/L maltose solution to the reactor from the liquid phase inlet 11 at the top end of the reactor at a flow rate of 1.5 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 87.2%, and a yield of the maltitol is 83.3%.

### Example 6:

The gas-liquid countercurrent trickle bed reactor was filled with 80 g of Raney nickel catalyst (with an average particle size of 3 mm), the reactor was heated up to 140 °C, the air inside the reactor was replaced and expelled with high-purity nitrogen, and then 50 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 7.5 MPa. Meanwhile, the liquid-phase metering pump was used to add 1.4 mol/L maltose solution to the reactor from the liquid phase inlet 11 at the top end of the reactor at a flow rate of 1.5 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 98.3%, and a yield of the maltitol is 94.5%.

### Example 7:

The gas-liquid countercurrent trickle bed reactor was filled with 100 g of Raney nickel catalyst (with an average particle size of 3 mm), the reactor was heated up to 125 °C, the air inside the reactor was replaced and expelled with high-purity nitrogen, and then 70 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 6 MPa. Meanwhile, the liquid-phase metering pump was used to add 1.4 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 87.6%, and a yield of the maltitol is 84.4%.

### Example 8:

The gas-liquid countercurrent trickle bed reactor was filled with 100 g of Raney nickel catalyst (with an average particle size of 3 mm), the reactor was heated up to 125 °C, the air inside the reactor was replaced and expelled with high-purity nitrogen, and then 70 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 7 MPa. Meanwhile, the liquid-phase metering pump was used to add 1.4 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 92.3%, and a yield of the maltitol is 90.0%.

### Example 9:

The gas-liquid countercurrent trickle bed reactor was filled with 100 g of Raney nickel catalyst (with an average particle size of 2.5 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 9 MPa. Meanwhile, the liquid-phase metering pump was used to add 0.6 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 99.4%, and a yield of the maltitol is 96.9%.

### Example 10:

The gas-liquid countercurrent trickle bed reactor was filled with 100 g of Raney nickel catalyst (with an average particle size of 2.5 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 9 MPa. Meanwhile, the liquid-phase metering pump was used to add 1.0 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 98.7%, and a yield of the maltitol is 95.3%.

### Example 11:

The gas-liquid countercurrent trickle bed reactor was filled with 100 g of Raney nickel catalyst (with an average particle size of 2.5 mm), the reactor was heated up to 130 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 9 MPa. Meanwhile, the liquid-phase metering pump was used to add 2 mol/L maltose solution to the reactor from the liquid phase inlet 11 at the top end of the reactor at a flow rate of 1 mL/min, so that the hydrogen gas and the maltose solution can pass through the catalyst bed 16 in the gas-liquid countercurrent manner for continuous hydrogenation reaction, and the maltose hydrogenation solution is obtained from the liquid phase outlet 14 at the bottom of the reactor.

A sample of the above maltose hydrogenation solution was analyzed by high-performance liquid chromatography (HPLC), and the results include that: a conversion rate of the maltose is 88.2%, and a yield of the maltitol is 86.1%.

### Example 12:

The gas-liquid countercurrent trickle bed reactor was filled with 100 g of Raney nickel catalyst (with an average particle size of 3 mm), the reactor was heated up to 130 °C, the air inside the reactor was replaced and expelled with high-purity nitrogen, and then 50 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 7 MPa. Meanwhile, the liquid-phase metering pump was used to add 1.6 mol/L glucose solution to the reactor from the liquid phase inlet 11 at the top end of the reactor at a flow rate of 1 mL/min, so that the hydrogen gas and the glucose solution can pass through the catalyst bed 16 in the gas-liquid countercurrent manner for continuous hydrogenation reaction, and the glucose hydrogenation solution is obtained from the liquid phase outlet 14 at the bottom of the reactor.

A sample of the above glucose hydrogenation solution was analyzed by high-performance liquid chromatography (HPLC), and the results include that: a conversion rate of the glucose is 99.1%, and a yield of the sorbitol is 98.0%.

### Example 13:

The gas-liquid countercurrent trickle bed reactor was filled with 90 g of Raney nickel catalyst (with an average particle size of 2 mm), the reactor was heated up to 125 °C, the air inside the reactor was replaced and expelled with high-purity nitrogen, and then 20 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 8 MPa. Meanwhile, the liquid-phase metering pump was used to add 1.4 mol/L mannose solution to the reactor from the liquid phase inlet 11 at the top end of the reactor at a flow rate of 1 mL/min, so that the hydrogen gas and the mannose solution can pass through the catalyst bed 16 in the gas-liquid countercurrent manner for continuous hydrogenation reaction, and the mannose hydrogenation solution is obtained from the liquid phase outlet 14 at the bottom of the reactor.

A sample of the above mannose hydrogenation solution was analyzed by high-performance liquid chromatography (HPLC), and the results include that: a conversion rate of the mannose is 99.7%, and a yield of the mannitol is 98.5%.

### Example 14:

The gas-liquid countercurrent trickle bed reactor was filled with 80 g of Raney nickel catalyst (with an average particle size of 2.5 mm), the reactor was heated up to 120 °C, the air inside the reactor was replaced and expelled with high-purity nitrogen, and then 30 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 6 MPa. Meanwhile, the liquid-phase metering pump was used to add 1.4 mol/L xylose solution to the reactor from the liquid phase inlet 11 at the top end of the reactor at a flow rate of 1 mL/min, so that the hydrogen gas and the xylose solution can pass through the catalyst bed 16 in the gas-liquid countercurrent manner for continuous hydrogenation reaction, and the xylose hydrogenation solution is obtained from the liquid phase outlet 14 at the bottom of the reactor.

A sample of the above xylose hydrogenation solution was analyzed by high-performance liquid chromatography (HPLC), and the results include that: a conversion rate of the xylose is 99.9%, and a yield of the xylitol is 98.7%.

### Example 15:

The gas-liquid countercurrent trickle bed reactor was filled with 80 g of Raney nickel catalyst (with an average particle size of 3.5 mm), the reactor was heated up to 130 °C, the air inside the reactor was replaced and expelled with high-purity nitrogen, and then 40 mL/min of hydrogen gas was introduced into the reactor from the gas phase inlet 13 at the lower end of the reactor and flowed out from the gas phase outlet 12 at the upper end of the reactor, and the pressure inside the reactor was controlled by the back-pressure valve at 8 MPa. Meanwhile, the liquid-phase metering pump was used to add 1.2 mol/L lactose solution to the reactor from the liquid phase inlet 11 at the top end of the reactor at a flow rate of 1 mL/min, so that the hydrogen gas and the lactose solution can pass through the catalyst bed 16 in the gas-liquid countercurrent manner for continuous hydrogenation reaction, and the lactose hydrogenation solution is obtained from the liquid phase outlet 14 at the bottom of the reactor.

A sample of the above lactose hydrogenation solution was analyzed by high-performance liquid chromatography (HPLC), and the results include that: a conversion rate of the lactose is 97.3%, and a yield of the lactitol is 95.5%.

### Comparative example 1:

A gas-liquid concurrent trickle bed reactor b was filled with 80 g of Raney nickel catalyst (with an average particle size of 3 mm) above a support frame 27, the reactor was heated up to 130 °C, the air inside the reactor was replaced and expelled with high-purity nitrogen, and then 30 mL/min of hydrogen gas was introduced into the reactor from a gas phase inlet 22 at the lower end of the reactor, and the pressure inside the reactor was controlled by the back-pressure valve at 8 MPa. Meanwhile, the liquid-phase metering pump was used to add 1.4 mol/L maltose solution to the reactor from a liquid phase inlet 21 at the top end of the reactor at a flow rate of 1 mL/min, so that after being mixed by a T-tube, the hydrogen gas and maltose solution can pass through an upper layer of quartz sand filler 24, a catalyst bed 25, and a lower layer of quartz sand filler 26 in succession for continuous hydrogenation reaction, and the maltose hydrogenation solution is obtained from a gas-liquid outlet 23 at the bottom of the reactor after the gas-liquid separation.

A sample of the above maltose hydrogenation solution was analyzed by high-performance liquid chromatography (HPLC), and the results include that: a conversion rate of the maltose is 73.1%, and a yield of the maltitol is 69.7%.

The above-described specific embodiments provide a detailed description of the technical solutions and beneficial effects of the present disclosure. It should be understood that the above description is only the most preferred embodiments of the present disclosure, and is not intended to limit the present disclosure. Any modifications, supplements, equivalent replacements, etc., made within the scope of the principles of the present disclosure shall be included in the scope of protection of the present disclosure.

## Claims

1. A method for continuous gas-liquid countercurrent preparing sugar alcohol, wherein the method comprises:
in a gas-liquid countercurrent trickle bed reactor, pouring a biomass sugar solution from a liquid phase inlet into the trickle bed reactor from top to bottom; and introducing hydrogen gas from a gas phase inlet to the trickle bed reactor from bottom to top, wherein the biomass sugar solution and the hydrogen gas pass through a catalyst bed in a gas-liquid countercurrent manner for hydrogenation reduction to obtain a biomass sugar alcohol solution.

2. The method of claim 1, wherein a catalyst includes Raney nickel, Raney copper, ruthenium carbon, or platinum carbon.

3. The method of claim 1 or claim 2, wherein an average particle size of the catalyst is in a range of 1 mm-6 mm.

4. The method of claim 1, wherein biomass sugar in the biomass sugar solution includes maltose, glucose, mannose, xylose, or lactose, and the sugar alcohol includes maltitol, sorbitol, mannitol, xylitol, or lactitol.

5. The method of claim 1, wherein a molar concentration of the biomass sugar solution is in a range of 0.6 mol/L -2.2 mol/L, and a feed flow rate of the biomass sugar solution is in a range of 0.5 mL/min-5 mL/min.

6. The method of claim 1, wherein a pressure of the hydrogen gas is in a range of 5 Mpa-10 MPa, and a flow rate of the hydrogen gas is in a range of10 sccm-100 sccm.

7. The method of claim 1, wherein a temperature of the trickle bed reactor is in a range of 100°C-140°C.

8. The method of claim 1, wherein a feed flow rate of the biomass sugar solution is in a range of 1 mL/min-2 mL/min, a pressure of the hydrogen gas is in a range of 6 Mpa-8 MPa, a flow rate of the hydrogen gas is in a range of 20 sccm-80 sccm, and a temperature of the trickle bed reactor is in a range of 120 °C-135°C.
